(19) Europäisches Patentamt
European Patent Office
Office européen des brevets

(11) Veröffentlichungsnummer: **0 073 060**
**A1**

(12) **EUROPÄISCHE PATENTANMELDUNG**

(21) Anmeldenummer: 82107787.2

(22) Anmeldetag: 25.08.82

(51) Int. Cl.³: C 07 D 487/04
A 61 K 31/505
//(C07D487/04, 239/00, 235/00)

(30) Priorität: 28.08.81 CH 5548/81
17.06.82 CH 3744/82

(43) Veröffentlichungstag der Anmeldung:
02.03.83 Patentblatt 83/9

(84) Benannte Vertragsstaaten:
AT BE CH DE FR GB IT LI LU NL SE

(71) Anmelder: F. HOFFMANN-LA ROCHE & CO.
Aktiengesellschaft

CH-4002 Basel(CH)

(72) Erfinder: Kienzle, Frank, Dr.
Tannwaldweg 9
CH-4113 Flüh(CH)

(74) Vertreter: Lederer, Franz, Dr. et al,
Patentanwälte Dr. Lederer Franz Meyer-Roxlau Reiner F.
Lucile-Grahn-Strasse 22
D-8000 München 80(DE)

(54) **Neue Imidazochinazolinderivate, ihre Herstellung und diese Derivate enthaltende Arzneimittel.**

(57) Die neuen Imidazochinazoline der Formel

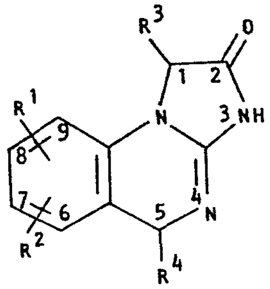

1

worin einer der Reste $R^1$ und $R^2$ Wasserstoff, Chlor, Brom, $C_{1-4}$-n-Alkyl oder $C_{1-4}$-n-Alkoxy und der andere $-N(R,R'')$ bedeutet, R und R″ $C_{1-4}$-n-Alkyl oder R zusammen mit R″ Tetra- oder Pentamethylen und $R^3$ und $R^4$ Wasserstoff oder Methyl bedeuten,

und deren Tautomere, sowie physiologisch verträgliche Säureadditionssalze solcher Verbindungen, hemmen die Aggregation der Blutplättchen bzw. die Magensekretion, bzw. sind kreislaufwirksam. Diese Verbindungen werden ausgehend von entsprechenden 4-Benzylimidazo[1,2-a]chinazolin-2(1H)-onen hergestellt, in denen die Benzylgruppe gegebenenfalls in ortho- oder para-Stellung durch $C_{1-4}$-Alkyl order $C_{1-4}$-Alkoxy substituiert ist.

EP 0 073 060 A1

Croydon Printing Company Ltd

F.Hoffmann-La Roche & Co.Aktiengesellschaft, Basel/Schweiz 0073060

- 1 -

25. Aug. 1982

RAN 4044/58

## Neue Imidazochinazolinderivate, ihre Herstellung und diese Derivate enthaltende Arzneimittel

Die vorliegende Erfindung betrifft Imidazochinazoline der Formel

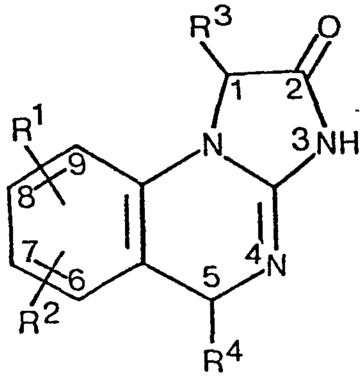

I

worin einer der Reste $R^1$ und $R^2$ Wasserstoff, Chlor, Brom, $C_{1-4}$-n-Alkyl oder $C_{1-4}$-n-Alkoxy und der andere -N(R,R") bedeutet, R und R" $C_{1-4}$-n-Alkyl oder R zusammen mit R" Tetra- oder Pentamethylen und $R^3$ und $R^4$ Wasserstoff oder Methyl bedeuten,

und deren Tautomere, sowie physiologisch verträgliche Säureadditionssalze solcher Verbindungen.

Diese Verbindungen sind neu und zeichnen sich durch wertvolle pharmakodynamische Wirkungen aus.

Mé/ 24.5.82

Die Erfindung betrifft ferner die besagten Verbindungen als pharmazeutische Wirkstoffe, sowie die Herstellung dieser Verbindungen, ferner Arzneimittel enthaltend eine Verbindung der Formel I oder ein pharmazeutisch anwendbares Salz davon, und die Herstellung solcher Arzneimittel, sowie die Verwendung von Verbindungen der Formel I oder von pharmazeutisch anwendbaren Salzen davon bei der Bekämpfung bzw. Verhütung von Krankheiten.

Der hier verwendete Ausdruck $C_{1-4}$-n-Alkyl bezieht sich auf Methyl, Aethyl und die geradkettigen Reste, Propyl und Butyl und der Ausdruck $C_{1-4}$-n-Alkoxy auf die den besagten Alkylresten entsprechenden Alkoxyresten.

Unter den Verbindungen der Formel I sind diejenigen bevorzugt, worin der Rest -N(R,R") sich in 6- oder 7-Stellung, insbesondere in 6-Stellung, befindet; ferner diejenigen worin R und R" $C_{1-4}$-n-Alkyl, insbesondere Methyl, bedeuten; schliesslich diejenigen, worin $R^3$ und $R^4$ Wasserstoff bedeuten.

Beispiele solcher bevorzugten Verbindungen sind:

7-Dimethylamino-4,5-dihydroimidazo[1,2-a]chinazolin-2(1H)-on,
deren Tautomere, sowie Säureadditionssalze dieser Verbindungen.

Beispiele physiologisch verträglicher Säureadditionssalze sind Mineralsäuresalze, insbesondere Hydrochloride, Hydrobromide, Sulfate und Phosphate; Salze mit organischen Sulfonsäuren, z.B. Alkyl- oder Arylsulfonate; und Carbonsäuresalze, wie Acetate, Fumarate, Oxalate und Citrate.

Die Verbindungen der Formel I können in verschiedenen tautomeren Formen vorliegen. Die Erfindung beschränkt sich daher nicht auf Verbindungen der oben dargestellten Formel I, sondern umfasst auch die Tautomeren, z.B. solche der

Formeln

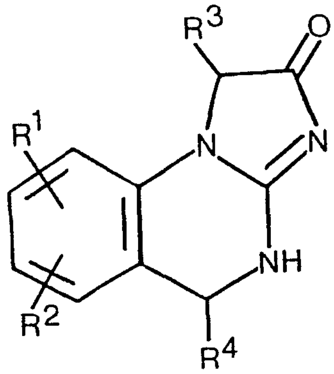

I-A

und

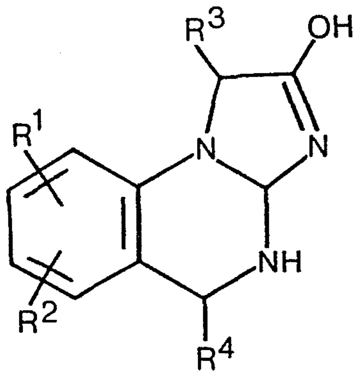

I-B

worin $R^1$-$R^4$ die obigen Bedeutungen haben.

Die Verbindungen der Formel I und deren Tautomeren, worin $R^3$ und/oder $R^4$ von Wasserstoff verschieden sind/ist, können weiterhin in Form von Racematen oder in optisch aktiver Form vorliegen, wobei alle diese Formen Gegenstand der Erfindung sind.

Die Verbindungen der Formel I und deren Tautomere, sowie die Salze solcher Verbindungen können erfindungsgemäss dadurch hergestellt werden, dass man eine Verbindung der Formel

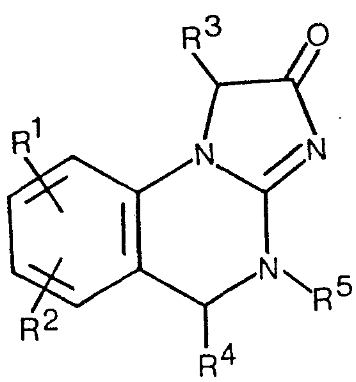

II

worin $R^1$-$R^4$ die obigen Bedeutungen haben und $R^5$ eine gegebenenfalls in ortho- oder para-Stellung durch $C_{1-4}$-Alkyl oder $C_{1-4}$-Alkoxy ringsubstituierte Benzylgruppe ist,

mit einer Säure behandelt, gewünschtenfalls eine erhaltene Verbindung der Formel I, worin einer der Reste $R^1$ und $R^2$ Wasserstoff ist, chloriert oder bromiert und eine erhaltene Verbindung der Formel I oder ein Tautomer davon in dieser Form oder in Form eines physiologisch verträglichen Säureadditionssalzes isoliert.

Als Säure verwendet man zweckmässigerweise eine Mineralsäure, wie Orthophosphorsäure. Die Umsetzung kann bei einer Temperatur zwischen Raumtemperatur und 170°C, vorzugsweise zwischen 100 und 150°C, vorzugsweise in Anwesenheit von Anisol, durchgeführt werden.

Die fakultative Chlorierung oder Bromierung wird zweckmässig dadurch bewerkstelligt, dass man eine Verbindung der Formel I, worin einer der Reste $R^1$ und $R^2$ Wasserstoff ist, bei einer Temperatur zwischen etwa 0 und 50°C, vorzugsweise bei Raumtemperatur, in Eisessig mit Chlor oder Brom, gegebenenfalls mit einem Katalysator wie $FeCl_3$, umsetzt.

Die Verbindungen der Formel II können durch Umsetzung einer Verbindung der Formel

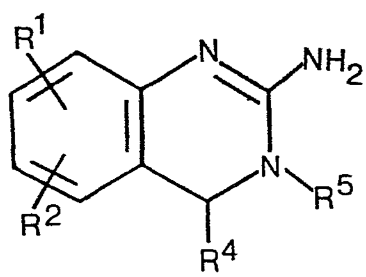

III

mit einem Ester der Formel

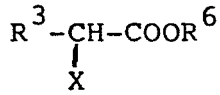

IV

worin $R^1$-$R^5$ die obigen Bedeutungen haben, $R^6$ $C_{1-4}$-Alkyl und X Chlor, Brom oder Jod, vorzugsweise Jod, bedeuten,

zweckmässigerweise in einem organischen Lösungsmittel, wie Dimethylformamid oder Acetonitril, bei einer Temperatur bis zur Rückflusstemperatur des Reaktionsgemisches, vorzugsweise in Anwesenheit einer anorganischen Base, wie Kaliumcarbonat, hergestellt werden.

Die Verbindungen der Formel III können, in Analogie zu dem in Chem. Pharm. Bull 28 (1980) 1357-1364 beschriebenen Verfahren zur Herstellung von 3-substituierten 2-Amino-3,4-dihydrochinazolinderivaten ausgehend von 2-Nitrobenzylchloriden, nach dem folgenden Reaktionsschema, worin $R^1$-$R^5$ die obigen Bedeutungen haben, hergestellt werden:

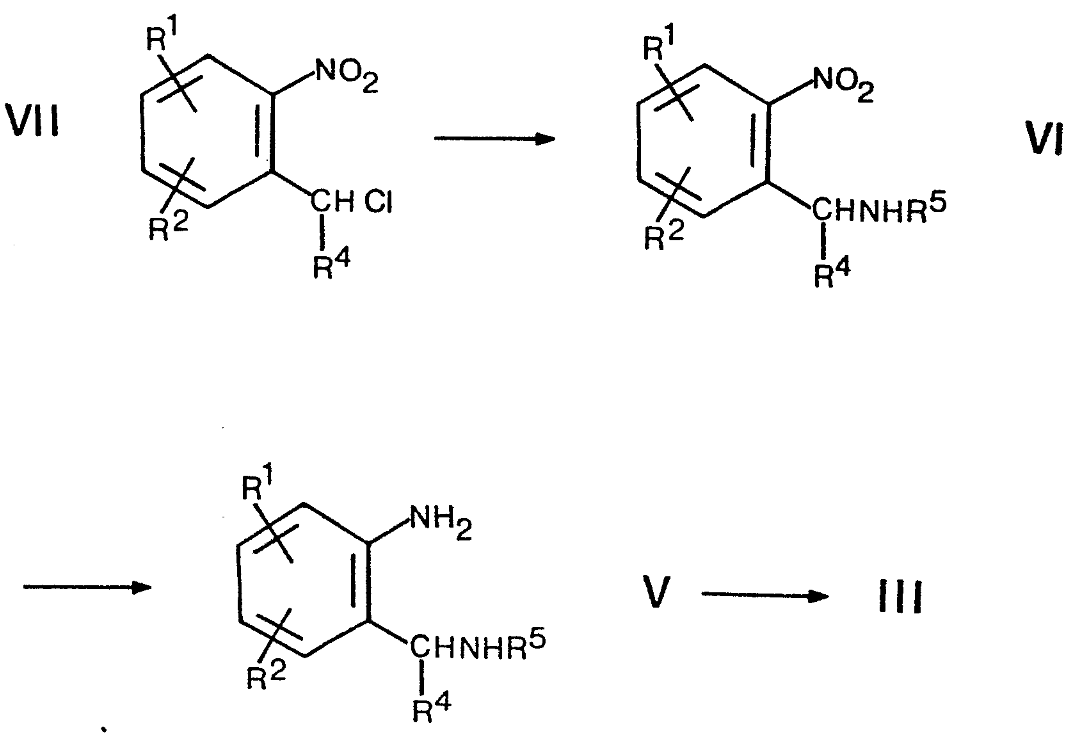
VII VI V III

In dieser Reaktion können an Stelle der Chloride der Formel VII die entsprechenden Bromide verwendet werden. Diese Chloride und Bromide können z.B. in Analogie zu der im Beispiel 1 beschriebenen Herstellung des α-Chlor-N,N-dimethyl-4-nitrotoluidins, durch Reduktion der entsprechenden 2-Nitrobenzoesäuren und Chlorierung bzw. Bromierung der so erhaltenen 2-Nitrobenzylalkohole hergestellt werden.

Die 2-Nitrobenzoesäuren sind bekannt oder können z.B. in Analogie zu der in der US Patentschrift 4 011 323 beschriebenen Herstellung der 5-Dimethylamino-2-nitrobenzoesäure hergestellt werden.

Die Verbindungen der Formel I, deren Tautomere und physiologisch verträgliche Salze solcher Verbindungen können als Heilmittel Verwendung finden. Sie hemmen z.B. die Aggregation der Blutplättchen und können daher zur Verhütung von Thrombosen verwendet werden. Ferner hemmen sie die Magensäuresekretion und können daher zur Behandlung von Magenulcera Verwendung finden. Ausserdem sind sie kreislaufwirksam. So weisen sie z.B. positiv inotrope Wirkung auf, ohne eine wesentliche Tachycardie zu verursachen und können zur Behandlung und Verhütung der Herzinsuffizienz verwendet werden. Eine gemeinsame vorteilhafte Eigenschaft der erfindungsgemässen Verbindungen ist ihre hohe Wasserlöslichkeit.

Die Verbindungen der Formel I und deren Tautomere können als Heilmittel z.B. in Form pharmazeutischer Präparate Verwendung finden, welche sie oder ihre Salze in Mischung mit einem für die enterale, perkutane oder parenterale Applikation geeigneten pharmazeutischen, organischen oder anorganischen inerten Trägermaterial, wie z.B. Wasser, Gelatine, Gummi arabicum, Milchzucker, Stärke, Magnesiumstearat, Talk, pflanzliche Oele, Polyalkylenglykole, Vaseline, usw. enthalten. Die pharmazeutischen Präparate können in fester Form, z.B. als Tabletten, Dragées, Suppositorien, Kapseln; in halbfester Form, z.B. als Salben; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes oder Puffer.

Sie können auch noch andere therapeutisch wertvolle Stoffe enthalten.

Die orale und die parenterale Verabreichung der erfindungsgemässen Verbindungen sind bevorzugt. Für den Erwachsenen kommen eine orale Tagesdosis von 0,1 bis 30 mg/kg und eine parenterale Tagesdosis von 0,01 bis 10 mg/kg in Frage. Für die Verhütung von Thrombosen beim Erwachsenen ist eine Tagesdosis von 1-10 mg angebracht und für die Behandlung von Herzinsuffizienz eine solche von 5-30 mg, jeweils unter Berücksichtigung der individuellen Bedürfnisse des Patienten und der Verabreichungsform.

Die aggregationshemmende Wirkung wurde nach der Aggregometer-Methode von BORN [Nature 194, 927 (1962)] und MICHAL und BORN [Nature 231, 220 (1971)] nachgewiesen. Die maximale Aggregationsgeschwindigkeit wurde als Versuchsparameter genommen und die effektive Konzentration ($EC_{50}$) aus Dosis-Wirkungskurven ermittelt.

Menschliches plättchenreiches Plasma wurde aus citriertem venösem Blut durch Zentrifugieren erhalten. Die Versuche wurden mit Suspensionen der Testsubstanzen in 0,9% NaCl durchgeführt. 0,18 ml Citratplasma wurden mit 10 µl Suspension der Testverbindungen versetzt und 10 Minuten bei 37°C inkubiert, worauf die Aggregation durch Zusatz von 10 µl einer Suspension von Kollagenfibrillen eingeleitet wurde.

Mit 7-Dimethylamino-4,5-dihydroimidazo[1,2-a]chinazolin-2(1H)-on-hydrochlorid wurde ein $EC_{50}$-Wert von 6,5 µM gefunden.

Zur Bestimmung der magensäuresekretionshemmenden Wirkung wurde die nachstehend beschriebene Versuchsanordnung verwendet: Weiblichen Ratten, welche 24 Stunden keine Nahrung, jedoch Wasser ad libitum erhalten hatten, wird unter leichter Aethernarkose gemäss Shay et al. (Gastroenterology, 5, 1945, 43) der Pylorus ligiert. Unmittelbar anschliessend wird den Tieren die zu prüfende Substanz intraduodenal verabreicht. Vier Stunden später tötet man die Tiere, bestimmt das Volumen und die Azidität ihres Magensaftes und vergleicht die erhaltenen Werte mit denjenigen von Kontrolltieren, welche gleich behandelt wurden, jedoch keine Testsubstanz erhalten hatten. Als ED 50 bezeichnet man diejenige Dosis der Testsubstanz, die bei den behandelten Tieren im Vergleich zu den Kontrolltieren eine 50%ige Verminderung von Volumen (ED 50-Volumen) bzw. Azidität (ED 50-Azidität) des Magensaftes bewirkt. Für das 7-Dimethylamino-4,5-dihydroimidazol[1,2-a]chinazolin-2(1H)-on-hydrochlorid wurden die folgenden Werte gefunden: ED 50-Volumen = 90 mg/kg i.d. und ED 50-Azidität = 65 mg/kg i.d.

Die positiv inotrope Wirkung wurde nach oraler Gabe der Prüfsubstanzen an wachen Schäferhunden gemessen. Die Tiere sind zu diesem Zweck mit einem implantierten Druck-Telemetrie-System ausgerüstet, wobei der Druckaufnehmer im linken Ventrikel fixiert ist. Der linksventrikuläre Druck wird über den implantierten Radiosender aus dem Tier gesendet und über ein geeignetes Antennen- und Empfängersystem empfangen, demoduliert und verstärkt. Durch Differenzierung des ansteigenden Schenkels des linksventrikulären Druckes (LVP) wird die maximale Druckanstiegsgeschwindigkeit ($dLVP/dt_{max}$) errechnet, was als Kontraktilitätsparameter gilt. Gleichzeitig wird die Herzfrequenz über einen Cardiotachographen aufgezeichnet. Unter Inotropie werden die prozentuale Veränderung (Δ%) von $dLVP/dt_{max}$ und die Wirkungsdauer in Stunden (Std) angegeben. Unter Tachycardie werden die prozentualen Veränderungen der Herzfrequenz (Δ%) nach Gabe der Prüfsubstanz und die Wirkungsdauer in Stunden (Std) angegeben. Für das 7-Dimethylamino-4,5-dihydroimidazo[1,2-a]chinazolin-2(1H)-on-hydrochlorid wurde bei einer Dosis von 10 mg/kg eine positiv inotrope Wirkung (Δ = +50%, 6 Stunden) ohne wesentliche Tachycardie (Δ = +36%, 6 Stunden) festgestellt.

In den nachstehenden Beispielen sind die Temperaturen in Grad Celsius angegeben.

Beispiel 1

Eine Lösung von 7 g 7-Dimethylamino-4,5-dihydro-4-(p-methoxybenzyl)-imidazo[1,2-a]chinazolin-2(1H)-on in 200 ml 85%iger Phosphorsäure und 10 ml Anisol wurden 24 Stunden bei 135° gerührt. Nach dem Abkühlen wurde mit Kalilauge auf pH 9 gestellt und das Produkt abfiltriert. Das Produkt, 7-Dimethylamino-4,5-dihydroimidazo[1,2-a]-chinazolin-2(1H)-on, wurde dann aus einem Gemisch von wässriger Salzsäure-Aethanol umkristallisiert und als Dihydrochloridmonohydrat vom Schmelzpunkt 268° (Zers.) isoliert.

Das Ausgangschinazolin kann wie folgt hergestellt werden:

a) Eine Lösung von 189 g 5-Dimethylamino-2-nitrobenzoesäure in 1,5 l 1,2-Dimethoxyäthan wurde mit 52 g Natriumborhydrid und dann tropfenweise bei 10° mit einer Lösung von 225 ml Bortrifluorid-ätherat in 500 ml 1,2-Dimethoxyäthan versetzt. Dann wurde 1 Stunde bei Raumtemperatur gerührt, vorsichtig auf Eis/Wasser gegossen und das Rohprodukt abfiltriert. Umkristallisation aus Essigester-Hexan gab reinen 5-Dimethylamino-2-nitrobenzylalkohol, Smp. 138-139°.

b) Eine Suspension von 78 g 5-Dimethylamino-2-nitrobenzylalkohol in 80 ml Dimethylformamid und 1 l Aether wurde tropfenweise bei 10° mit 60 ml Thionylchlorid versetzt. Nach 4 Stunden bei 10° wurde auf Wasser gegossen, vorsichtig mit Natriumbicarbonat neutralisiert und mit Essigester extrahiert. Nach dem Abdampfen des Essigesters wurde der Rückstand in kaltem Methanol digeriert und abfiltriert. Ausbeute an α-Chlor-N,N-dimethyl-4-nitro-o-toluidin, 84 g, Smp. 120° (Zers.).

c) Eine Lösung von 79 g α-Chlor-N,N-dimethyl-4-nitro-o-toluidin in 1,2 l Aethanol wurde mit 100 ml 4-Methoxybenzylamin und 140 ml Triäthylamin versetzt und 4 Stunden unter Rückfluss gekocht. Dann wurde das Lösungsmittel abgedampft, der Rückstand in 10% Natriumcarbonatlösung aufgeschlämmt und mit Essigester extrahiert. Nach dem Abdampfen wurde der Rückstand aus Essigester/Hexan kristallisiert. Smp. des reinen 3-Dimethylamino-4'-methoxy-6-nitro-dibenzylamins, 82-83°.

d) Eine Lösung von 77 g 3-Dimethylamino-4'-methoxy-6-nitro-dibenzylamin in 1100 ml Aethanol und 70 ml Triäthylamin wurde in Anwesenheit von Raney-Nickel hydriert. Nach Filtration und Eindampfen wurde das Produkt, 3-Dimethylamino-4'-methoxy-6-aminodibenzylamin, als Oel erhalten.

e) Eine Lösung von 63 g 3-Dimethylamino-4'-methoxy-6-amino-dibenzylamin in 900 ml Dioxan wurde mit 23,5 g Bromcyan versetzt und 20 Stunden bei Raumtemperatur gerührt. Dann wurden 150 ml Diäthyläther dazugegeben. Die Lösung wurde 4 Stunden unter Rückfluss gekocht. Nach dem Abkühlen wurde vom festen Rohprodukt abdekantiert und das Rohprodukt aus Methanol umkristallisiert. Ausbeute: 32 g 2-Amino-6-dimethylamino-3,4-dihydro-3-(p-methoxybenzyl)-chinazolin, Smp. 175-180° (als Hydrobromid).

f) Eine Lösung von 19,5 g 2-Amino-6-dimethylamino-3,4-dihydro-3-(p-methoxybenzyl)-chinazolin in 300 ml Dimethylformamid wurde mit 90 g Kaliumcarbonat und 12,9 g Jodessigsäureäthylester versetzt und 20 Stunden bei Raumtemperatur, gefolgt von 5 Stunden bei 85° gerührt. Dann wurde abgekühlt, auf Eis/Wasser gegossen und das Rohprodukt abfiltriert. Reines 7-Dimethylamino-4,5-dihydro-4-(p-methoxybenzyl)-imidazo[1,2-a]chinazolin-2(1H)-on vom Schmelzpunkt 196-200° wurde durch Umkristallisation aus Methanol erhalten.

Beispiel 2

In zu Beispiel 1 analoger Weise wird aus 7-Dimethylamino-4,5-dihydro-4-(p-methoxybenzyl)-6-methylimidazo-[1,2-a]chinazolin-2(1H)-on, Smp. 245-248° (Zers.), das 7-Dimethylamino-4,5-dihydro-6-methylimidazo[1,2-a]chinazolin-2(1H)-on hergestellt, Smp. des Dihydrochlorids nach Kristallisation aus Aethanol 275-276° (Zers.).

Das Ausgangschinazolin wird in zu Beispiel 1 analoger Weise aus 5-Dimethylamino-6-methyl-2-nitrobenzylalkohol hergestellt. Letzterer kann wie folgt hergestellt werden:

1 Mol 2,6-Dichlor-3-nitrotoluol wurde in 1 l DMSO mit 1 Mol Kupfer(I)cyanid 5 Stunden bei 150° gerührt. Dann wurde das Gemisch abgekühlt, auf Wasser gegossen und mit Essigester extrahiert. Die organische Phase wurde eingedampft und der Rückstand auf Kieselgel chromatographisch gereinigt. Man erhält 2-Methyl-3-chlor-6-nitrobenzoesäurenitril (Ausbeute 60%), Smp. 97-98°.

142 g dieses Nitrils wurden in 1,4 l 80% Schwefelsäure 4 Stunden bei 100° gerührt, dann wurde abgekühlt und auf Wasser gegossen, und das rohe Säureamid (Smp. 155-157°) wurde abfiltriert. Dieses wurde in 600 ml 60% Schwefelsäure gelöst und bei 90° wurde eine Lösung von 81 g Kaliumnitrit in 100 ml Wasser zugetropft. Dann wurde abgekühlt, mit Wasser verdünnt und die ausfallende 3-Chlor-2-methyl-6-nitrobenzoesäure, Smp. 130-133° wurde abfiltriert.

Eine Lösung von 171 g dieser Säure wurde in 1 l 30%igen methanolischem Dimethylamin 20 Stunden im Autoklav bei 100° gerührt. Dann wurde das Gemisch abgekühlt, auf Wasser gegossen, mit Essigsäure angesäuert und mit Essigsäureäthylester extrahiert. Die organische Phase wurde eingedampft. Die rohe, teilweise feste 2-Nitro-5-dimethylamino-6-methylbenzoesäure (143 g) wurde in 1,5 ml Monoglyme gelöst und mit 33,4 g Natriumborhydrid versetzt. Zu

der Mischung wurden dann 143 ml Bortrifluorid-ätherat getropft und es wurde 20 Stunden gerührt. Dann wurde abgedampft, mit Wasser verdünnt und mit Essigsäureäthylester extrahiert. Die organische Phase wurde getrocknet, eingedampft und auf einer Kieselgelsäule chromatographiert.

46 g reines 2-Nitro-5-dimethylamino-6-methylbenzylalkohol, Smp. 42-43° wurden durch Umkristallisation aus Aether-Hexan erhalten.

Beispiel 3

In zu Beispiel 1 analoger Weise wird aus 6-Dimethylamino-4-(p-methoxybenzyl)-4,5-dihydroimidazo[1,2-a]chinazolin-2-(1H)-on das 6-Dimethylamino-4,5-dihydroimidazo-[1,2-a]chinazolin-2(1H)-on hergestellt.

Beispiel 4

In zu Beispiel 1 analoger Weise wird aus 6-Dimethylamino-1-methyl-4-(p-methoxybenzyl)-4,5-dihydroimidazo-[1,2-a]chinazolin-2(1H)-on das 6-Dimethylamino-1-methyl-4,5-dihydroimidazo[1,2-a]chinazolin-2(1H)-on hergestellt.

Beispiel 5

In zu Beispiel 1 analoger Weise wird aus 6-Dimethylamino-7-methyl-4-(p-methoxybenzyl)-4,5-dihydroimidazo-[1,2-a]chinazolin-2(1H)-on das 6-Dimethylamino-7-methyl-4,5-dihydroimidazo[1,2-a]chinazolin-2(1H)-on hergestellt.

Beispiel 6

In zu Beispiel 1 analoger Weise wird aus 7-Chlor-6-dimethylamino-4-(p-methoxybenzyl)-4,5-dihydroimidazo[1,2-a]-chinazolin-2(1H)-on das 7-Chlor-6-dimethylamino-4,5-dihydroimidazo[1,2-a]chinazolin-2(1H)-on hergestellt.

Beispiel 7

In zu Beispiel 1 analoger Weise wird aus 6-Dimethylamino-7-methoxy-4-(p-methoxybenzyl)-4,5-dihydroimidazo-[1,2-a]chinazolin-2(1H)-on das 6-Dimethylamino-7-methoxy-4,5-dihydroimidazo[1,2-a]chinazolin-2(1H)-on hergestellt.

Beispiel 8

In zu Beispiel 1 analoger Weise wird aus 6-Chlor-7-dimethylamino-4-(p-methoxybenzyl)-4,5-dihydroimidazo[1,2-a]-chinazolin-2(1H)-on das 6-Chlor-7-dimethylamino-4,5-dihydroimidazo[1,2-a]chinazolin-2(1H)-on hergestellt.

Beispiel 9

In zu Beispiel 1 analoger Weise wird aus 7-Dimethylamino-1-methyl-4-(p-methoxybenzyl)-4,5-dihydroimidazo-[1,2-a]chinazolin-2(1H)-on das 7-Dimethylamino-1-methyl-4,5-dihydroimidazo[1,2-a]chinazolin-2(1H)-on hergestellt.

Beispiel 10

In zu Beispiel 1 analoger Weise wird aus 6-Chlor-7-dimethylamino-1-methyl-4-(p-methoxybenzyl)-4,5-dihydroimidazo[1,2-a]chinazolin-2(1H)-on das 6-Chlor-7-dimethylamino-1-methyl-4,5-dihydroimidazo[1,2-a]chinazolin-2(1H)-on hergestellt.

## Beispiel 11

In üblicher Weise werden Tabletten folgender Zusammensetzung hergestellt:

| | |
|---|---|
| 7-Dimethylamino-4,5-dihydroimidazo[1,2-a]-chinazolin-2(1H)-on-hydrochlorid | 185,0 mg |
| Milchzucker | 15,0 mg |
| Maisstärke | 37,5 mg |
| Wasserlösliches Polyvinylpyrrolidon | 10,0 mg |
| Magnesiumstearat | 2,5 mg |
| Gesamtgewicht pro Tablette | 250,0 mg |

## Beispiel 12

In üblicher Weise werden Gelatinesteckkapseln folgender Zusammensetzung hergestellt:

| | |
|---|---|
| 7-Dimethylamino-4,5-dihydroimidazo[1,2-a]-chinazolin-2(1H)-on-hydrochlorid | 200,0 mg |
| Wasserlösliches Polyvinylpyrrolidon | 2,0 mg |
| Maisstärke | 43,0 mg |
| Talk | 4,5 mg |
| Magnesiumstearat | 0,5 mg |
| Gesamtgewicht pro Kapsel | 250,0 mg |

## Beispiel 13

In üblicher Weise wird eine Injektionslösung folgender Zusammensetzung hergestellt:

| | |
|---|---|
| 7-Dimethylamino-4,5-dihydroimidazo[1,2-a]-chinazolin-2(1H)-on-hydrochlorid | 115,0 mg |
| Glycerinformal | 2,4 ml |
| Wasser | 4,0 ml |

Patentansprüche

1. Imidazochinazoline der Formel

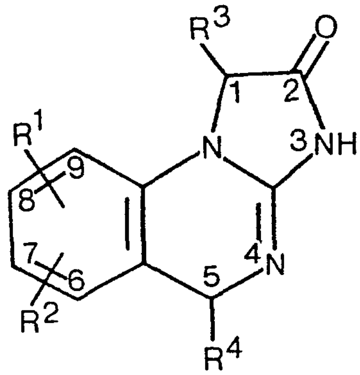

I

worin einer der Reste $R^1$ und $R^2$ Wasserstoff, Chlor, Brom, $C_{1-4}$-n-Alkyl oder $C_{1-4}$-n-Alkoxy und der andere -N(R,R") bedeutet, R und R" $C_{1-4}$-n-Alkyl oder R zusammen mit R" Tetra- oder Pentamethylen und $R^3$ und $R^4$ Wasserstoff oder Methyl bedeuten,
und deren Tautomere, sowie physiologisch verträgliche Säureadditionssalze solcher Verbindungen.

2. Verbindungen nach Anspruch 1, worin einer der Reste $R^1$ und $R^2$ Wasserstoff, Chlor oder Brom und der andere -N(R,R") bedeutet und R, R", $R^3$ und $R^4$ die gleiche Bedeutung wie in Anspruch 1 haben.

3. Verbindungen nach Anspruch 1 oder 2, worin der Rest -N(R,R") sich in 6- oder 7-Stellung, insbesondere in 6-Stellung, befindet.

4. Verbindungen nach Anspruch 1, 2 oder 3, worin R und R" $C_{1-4}$-n-Alkyl, insbesondere Methyl, bedeuten.

5. Verbindungen nach einem der Ansprüche 1-4, worin $R^3$ und $R^4$ Wasserstoff bedeuten.

6. 7-Dimethylamino-4,5-dihydroimidazo[1,2-a]chinazolin-2(1H)-on und dessen Tautomere, sowie physiologisch verträgliche Säureadditionssalze solcher Verbindungen.

7. Imidazochinazoline nach einem der Ansprüche 1-6 als pharmazeutische Wirkstoffe.

8. Imidazochinazoline nach einem der Ansprüche 1-6 als die Blutplättchenaggregation bzw. die Magensäuresekretion hemmende Wirkstoffe bzw. als kreislaufwirksame Mittel.

9. Verfahren zur Herstellung von Imidazochinazolinen der Formel I in Anspruch 1 und von deren Tautomeren, sowie von physiologisch verträglichen Säureadditionssalzen solcher Verbindungen, dadurch gekennzeichnet, dass man eine Verbindung der Formel

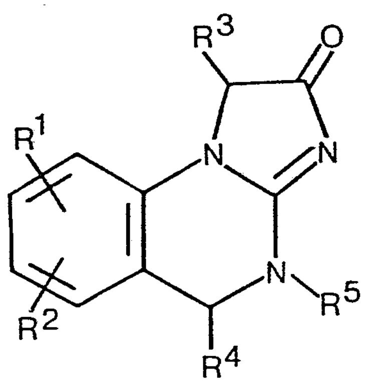

II

worin $R^1$-$R^4$ die in Anspruch 1 gegebenen Bedeutungen haben und $R^5$ eine gegebenenfalls in ortho- oder para-Stellung durch $C_{1-4}$-Alkyl oder $C_{1-4}$-Alkoxy ringsubstituierte Benzylgruppe bedeutet,

mit einer Säure umsetzt, gewünschtenfalls eine erhaltene Verbindung der Formel I, worin einer der Reste $R^1$ und $R^2$ Wasserstoff ist, chloriert oder bromiert und eine erhaltene Verbindung der Formel I oder ein Tautomer davon in dieser Form oder in Form eines physiologisch verträglichen Säureadditionssalzes isoliert.

10. Arzneimittel enthaltend ein Imidazochinazolin nach einem der Ansprüche 1-6.

11. Arzneimittel nach Anspruch 10 zur Hemmung der Blutplättchenaggregation bzw. der Magensäuresekretion bzw. kreislaufwirksames Arzneimittel.

***

Patentansprüche für Oesterreich

1. Verfahren zur Herstellung von Imidazochinazolinen der Formel

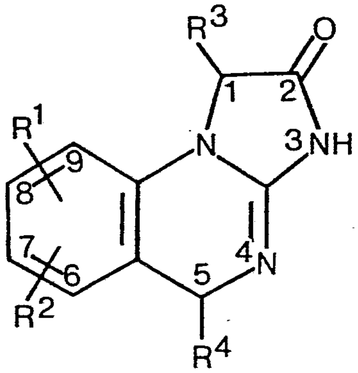

I

worin einer der Reste $R^1$ und $R^2$ Wasserstoff, Chlor, Brom, $C_{1-4}$-n-Alkyl oder $C_{1-4}$-n-Alkoxy und der andere -N(R,R") bedeutet, R und R" $C_{1-4}$-n-Alkyl oder R zusammen mit R" Tetra- oder Pentamethylen und $R^3$ und $R^4$ Wasserstoff oder Methyl bedeuten,

und deren Tautomere, sowie physiologisch verträgliche Säureadditionssalze solcher Verbindungen, dadurch gekennzeichnet, dass man eine Verbindung der Formel

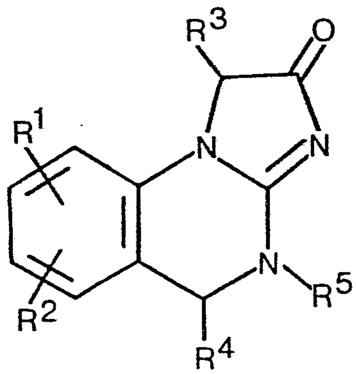

II

worin $R^1$-$R^4$ die in Anspruch 1 gegebenen Bedeutungen haben und $R^5$ eine gegebenenfalls in ortho- oder para-Stellung durch $C_{1-4}$-Alkyl oder $C_{1-4}$-Alkoxy ringsubstituierte Benzylgruppe bedeutet,

mit einer Säure umsetzt, gewünschtenfalls eine erhaltene

Verbindung der Formel I, worin einer der Reste $R^1$ und $R^2$ Wasserstoff ist, chloriert oder bromiert und eine erhaltene Verbindung der Formel I oder ein Tautomer davon in dieser Form oder in Form eines physiologisch verträglichen Säureadditionssalzes isoliert.

2. Verfahren nach Anspruch 1 zur Herstellung von Imidazochinazolinen der Formel I, worin einer der Reste $R^1$ und $R^2$ Wasserstoff, Chlor, Brom und der andere -N(R,R") bedeutet und R, R", $R^3$ und $R^4$ die gleiche Bedeutung wie in Anspruch 1 haben, und deren Tautomere, sowie physiologisch verträgliche Säureadditionssalze solcher Verbindungen.

3. Verfahren nach Anspruch 1 zur Herstellung von Imidazochinazolinen der Formel I, worin der Rest -N(R,R") sich in 6- oder 7-Stellung, insbesondere in 6-Stellung, befindet, und deren Tautomere, sowie physiologisch verträgliche Säureadditionssalze solcher Verbindungen.

4. Verfahren nach Anspruch 1 oder 2 zur Herstellung von Imidazochinazolinen der Formel I, worin R und R" $C_{1-4}$-n-Alkyl, insbesondere Methyl bedeutet, und deren Tautomere, sowie physiologisch verträgliche Säureadditionssalze solcher Verbindungen.

5. Verfahren nach einem der Ansprüche 1-3 zur Herstellung von Imidazochinazolinen der Formel I, worin $R^3$ und $R^4$ Wasserstoff bedeuten, und deren Tautomere, sowie physiologisch verträgliche Säureadditionssalze solcher Verbindungen.

6. Verfahren nach Anspruch 1 zur Herstellung des 7-Dimethylamino-4,5-dihydroimidazo[1,2-a]chinazolin-2(1H)-ons und dessen Tautomerer, sowie physiologisch verträglicher Säureadditionssalze solcher Verbindungen.

Europäisches Patentamt

**0073060**

Nummer der Anmeldung

## EUROPÄISCHER RECHERCHENBERICHT

EP 82 10 7787

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl. 3) |
|---|---|---|---|
| A | CHEMICAL ABSTRACTS, Band 95, Nr. 15, 12. Oktober 1981, Seite 682, Nr. 132958q, Columbus, Ohio, USA & JP - A - 81 29591 (DAIICHI SEIYAKU CO., LTD.) 24. März 1981 * Zusammenfassung * | 1,10 | C 07 D 487/04<br>A 61 K 31/505//<br>(C 07 D 487/04<br>C 07 D 239/00<br>C 07 D 235/00 ) |
| P,A | EP-A-0 046 267 (HOFFMANN-LA ROCHE) * Ansprüche 1,13 * | 1,10 | |
| | | | **RECHERCHIERTE SACHGEBIETE (Int. Cl. 3)** |
| | | | C 07 D 487/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 29-11-1982 | ALFARO I. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82